# EUROPÄISCHE PATENTSCHRIFT

(11) **EP 2 802 531 B1**
(45) Veröffentlichungstag und Bekanntmachung des Hinweises auf die Patenterteilung: **12.12.2018**
(21) Anmeldenummer: 13700262.2
(22) Anmeldetag: 02.01.2013
(51) Int. Cl.: C01B 25/38, C01B 25/40, H01M 4/58, H01M 10/0525

(54) **KONDENSIERTE EISEN (IIII) PHOSPHATE**
CONDENSED IRON (III) PHOSPHATE
PHOSPHATE DE FER(III) CONDENSÉ

(30) Priorität: 10.01.2012 DE 102012100128
(43) Veröffentlichungstag der Anmeldung: 19.11.2014
(73) Patentinhaber: Chemische Fabrik Budenheim KG, 55257 Budenheim (DE)
(72) Erfinder: BÜHLER, Gunnar, 56645 Nickenich (DE); STAY, Manola, 33110 Mersin (TR)
(74) Vertreter: WSL Patentanwälte Partnerschaft mbB
(86) Internationale Anmeldenummer: PCT/EP2013/050011
(87) Internationale Veröffentlichungsnummer: WO 2013/110476

(56) Entgegenhaltungen:
- WO-A1-87/06433
- DE-A1-102007 049 757
- DE-A1-102009 001 204
- REIFF W M ET AL: "Synthetic iron III phosphates: Topotactic transformation of Fe2(P2O7)(HPO4) to Fe4(P2O7)3, a more condensed magnetic structure", HYPERFINE INTERACTIONS, SPRINGER, Bd. 53, Nr. 1-4, 1. Juli 1990 (1990-07-01) , Seiten 403-408, XP008161585, ISSN: 0304-3843, DOI: 10.1007/BF02101074

## Beschreibung

### Gegenstand der Erfindung

Die Erfindung betrifft kondensiertes Eisen(III)phosphat der allgemeinen Formel Fe₍ₙ₊₂₎(PₙO₃ₙ₊₁)₃ · xH₂O mit n ≥ 2 und x ≤ 9 sowie ein Verfahren zu dessen Herstellung. Das kondensierte Eisen(III)phosphat eignet sich aufgrund seiner Eigenschaften unter anderem zur Verwendung als Lebensmitteladditiv zur Mineralstoffanreicherung.

### Hintergrund der Erfindung

Eisen(III)pyrophosphat (FePP) wird unter anderem als Eisenquelle im Bereich der Ernährung von Menschen und Tieren sowie bei der Düngung von Pflanzen eingesetzt. Beim Einsatz in der Nahrung von Menschen und Tieren hat Eisen(III)pyrophosphat den Vorteil, dass sein Einfluss auf die sensorischen Eigenschaften des Nahrungsmittels normalerweise vernachlässigbar ist.

Eisenmangel betrifft etwa ein Viertel der Erdbevölkerung und kann weitgehende Folgen haben, da Eisen in Organismen für eine Vielzahl physiologischer Funktionen benötigt wird, z.B. Sauerstofftransport von den Lungen zum Gewebe, Elektronentransport in Zellen und als Cofaktor für enzymatische Reaktionen.

Die Herstellung von Eisen(III)pyrophosphat (FePP) nach dem Stand der Technik erfolgt im Allgemeinen durch Kationen-Anionen-Austausch unter Ausnutzung des geringen Löslichkeitsproduktes von FePP. Dazu wird ein wasserlösliches Eisensalz, meist Eisensulfat, Eisenchlorid, Eisencitrat o. ä., mit einem Alkalidiphosphat, wie beispielsweise Tetranatriumpyrophosphat (TSPP) oder Dinatriumdihydrogenpyrophosphat (saures Natriumpyrophosphat; SAPP) in wässriger Lösung umgesetzt. Werden Fe(II)-Salze verwendet, so muss eine Oxidation zum Fe(III) durch Zusatz eines geeigneten Oxidationsmittels erfolgen. Die Einleitung bzw. Vervollständigung der Fällung des FePP aus der wässrigen Lösung erfolgt häufig durch Kontrolle des pH-Wertes im Bereich von etwa 3.

Nach der Fällung wird der Feststoff üblicherweise von der Lösung z. B. durch Filtration abgetrennt. Wird das FePP beispielsweise durch Umsetzung von Eisenchlorid oder Eisensulfat mit TSPP oder SAPP hergestellt, entstehen äquimolare Mengen an NaCl oder Na₂SO₄. Solche und andere Fremdsalze sind in der Regel unerwünscht und sollen nicht im Endprodukt verbleiben, da sie bei der späteren Anwendung des Produktes stören können, zum Beispiel durch farbliche Veränderung, Beeinflussung des Geschmacks, Verklumpen oder unerwünschte chemische Reaktionen mit anderen Formulierungskomponenten. Um unerwünschte Kationen und Anionen zu entfernen, muss der erhaltene Filterkuchen daher intensiv gewaschen werden. Nach der Trocknung wird FePP als bräunlich bis gelb-weißes Pulver erhalten, je nach Ausmaß und Art der Verunreinigungen mit Fremdionen.

Nach den bekannten Herstellungsmethoden erhält man FePP verschiedener Hydratstufen Fe₄(P₂O₇)₃ · xH₂O, wobei die isolierten Feststoffe amorph anfallen und daher strukturell über röntgenographische Methoden nicht bezüglich der chemischen Summenformel charakterisierbar bzw. identifizierbar sind. Zur Charakterisierung können jedoch die Gehalte der Hauptkonstituenten Fe, P₂O₅ und H₂O (über den Glühverlust GV) bestimmt werden. Zudem kann mittels IR-Spektroskopie das Vorliegen einer Pyrophosphat-Bande anhand einer Schwingung der O₃P-O-PO₃ Gruppe) festgestellt werden.

Der US-amerikanische FCC (Food Chemical Codex), eine Sammlung international anerkannter Monographie-Standards und Prüfverfahren zur Bestimmung der Reinheit und Qualität von Lebensmittelchemikalien, orientiert sich bezüglich der Vorgaben für Eisen(III)pyrophosphat (Ferric Pyrophosphate, FePP) an einer Verbindung der Formel Fe₄(P₂O₇)₃ · 9 H₂O, welche bezogen auf die ideal reine Verbindung folgende Gehalte (in Gewichts-%) der einzelnen Konstituenten aufweisen soll, wobei die Gehalte wie üblich als Fe₂O₃, P₂O₅ und H₂O (= Glühverlust GV) ausgedrückt werden:

| Fe₄(P₂O₇)₃ · 9 H₂O | |
|---|---|
| Fe₂O₃ : | 35,2 % |
| P₂O₅ : | 46,9 % |
| H₂O (GV) : | 17,9 % |

Der Gehalt an Eisen (Fe) beträgt 24,6 %. Als Bestimmungsgröße des FePP-Gehaltes in einem Produkt, welches herstellungsbedingt nicht nur reines FePP enthält, sondern auch Orthophosphat und/oder höher kondensierte Phosphatformen, kann zweckmäßig das Verhältnis von Fe:P₂O₅ herangezogen werden, da dieses Verhältnis nicht dem Einfluss verschiedener Anteile an Hydratwasser und oberflächengebundener Feuchtigkeit unterliegt. Das Fe:P₂O₅ Verhältnis eines idealen FePP der Formel Fe₄(P₂O₇)₃ · 9 H₂O beträgt demnach 0,525.

Gemäß FCC muss ein FePP folgender Spezifikation genügen:

| | |
|---|---|
| Fe : | 24,0 - 26,0 % |
| GV : | ≤ 20,0 % |
| As : | ≤ 3 mg/kg |
| Pb : | ≤ 4 mg/kg |
| Hg : | ≤ 3 mg/kg |

Der P₂O₅-Gehalt eines zum FCC konformen FePP ist demnach bislang nicht definiert. Bei der Herstellung eines zum FCC konformen FePP besteht daher die Möglichkeit, durch signifikante Variation des Glühverlustes (GV) in Bereiche deutlich unter die theoretischen 17,9 %, was spezifikationsgemäß tolerierbar ist, die Forderung von mindestens 24 % Fe einzuhalten. Je mehr sich der GV vom theoretischen Wert von 17,9 % des reinen FePP entfernt, desto geringer ist die Wahrscheinlichkeit, dass es sich bei der vorliegenden Verbindung um ein FePP mit den nach dem FCC angedachten Eigenschaften handelt. Ein geringer GV erfordert bei der Herstellung des Produktes einen hohen Energieverbrauch und hat eine mit abnehmendem GV deutlich zunehmende gelb-braune Färbung des Produktes zur Folge, was in vielen Anwendungsfällen eine unerwünschte Produkteigenschaft ist.

Bei einem Mindestgehalt an Fe von 24 % gemäß FCC und dem maximal zulässigen GV von 20 % ergibt sich bei dem entsprechenden Fe:P₂O₅-Verhältnis für reines FePP von 0,525 ein Produkt, das rechnerisch einen Mindestgehalt von 98 % FePP aufweist.

Bei einem Höchstgehalt an Fe von 26 % gemäß FCC und dem maximal zulässigen GV von 20 % ergibt sich ein Produkt, das rechnerisch 42,9 % P₂O₅ enthält, was nur einen Gehalt von höchstens 81,1 % FePP erlaubt. Folglich müssen bei einer solchen Konstellation zwangsläufig auch Verbindungen vorliegen, die nicht FePP entsprechen.

Die DE 102009 001 204 beschreibt unter anderem die Herstellung einer phosphorsauren Fe²⁺-Lösung, wobei oxidische Verbindungen des Eisens unterschiedlichster Art zusammen mit elementarem Eisen in wässrigen Lösungen von Orthophosphorsäure verschiedener Konzentrationen umgesetzt werden. Die Lösung enthält ausschließlich verdünnte wässrige Phosphorsäure und gelöste Fe²⁺-Ionen.

W.M. Reiff et al., "Synthetic iron III phosphates: Topotactic transformation of Fe2(P2O7)(HPO4) to Fe4(P2O7)3, a more condensed magnetic structure", Hyperfine interactions, Springer, Bd. 53, Nr. 1-4, 1. Juli 1990, Seiten 403-408, beschreiben einen Prozess zur Herstellung von Fe₄(P₂O₇)₃-Kristallen, in dem Fe₂O₃ in einem Goldrohr bei 700°C und 3 kbar mit H₃Po₄ reagiert. Das erhaltene Fe₂(P₂O₇)(HPO₄) wird über 800°C erhitzt und in Fe₄(P₂O₇)₃ überführt, welches als kristallines Produkt mit orthorhombischer Einheitszelle vorliegt.

### Aufgabe

Die Aufgabe der Erfindung bestand in der Bereitstellung neuer kondensierter Eisen(III)phosphate, die sich unter anderem durch einfache Herstellbarkeit in hoher Reinheit auszeichnen, und eines neuen Verfahrens zu deren Herstellung sowie deren Verwendung.

### Beschreibung der Erfindung

Die erfindungsgemäße Aufgabe wird gelöst durch ein Verfahren zur Herstellung von kondensiertem Eisen(III)phosphat der allgemeinen Formel Fe₍ₙ₊₂₎(PₙO₃ₙ₊₁)₃ · xH₂O mit n ≥ 2 und x ≤ 9, bei dem man
a) eine Fe²⁺-Ionen enthaltende wässrige Lösung herstellt, indem man Eisen(II)-, Eisen(III)- oder gemischte Eisen(II,III)-Verbindungen, ausgewählt unter Hydroxiden, Oxiden, Oxidhydroxiden, Oxidhydraten, Carbonaten und Hydroxidcarbonaten, zusammen mit elementarem Eisen in ein Phosphorsäure enthaltendes wässriges Medium einbringt, wobei Fe²⁺-Ionen in Lösung gebracht werden und Fe³⁺ mit elementarem Fe (in einer Komproportionierungsreaktion) zu gelöstem Fe²⁺ umgesetzt werden,
b) gegebenenfalls vorhandene Feststoffe von der phosphorsauren wässrigen Fe²⁺-Lösung abtrennt,
c) zu der phosphorsauren wässrigen Fe²⁺-Lösung ein Oxidationsmittel zugibt, um Eisen(II) in der Lösung zu oxidieren, wobei die Oxidationsbedingungen so gewählt werden, dass keine Eisen(III)phosphate ausgefällt werden, indem man die Temperatur der Reaktionslösung während der Zugabe des Oxidationsmittels durch Kühlung der Reaktionslösung und/oder durch Einstellen der Zugabegeschwindigkeit des Oxidationsmittels im Bereich von 10°C bis ≤ 60°C hält,
d) nach Abschluss der Oxidationsreaktion der erhaltenen wässrigen Fe³⁺-Lösung Polyphosphate in der Form von Polyphosphorsäure oder ihrer Salze als Feststoffe oder als wässrige Lösung zugibt, wobei kondensiertes Eisen(III)phosphat als Feststoff ausgefällt wird,
e) das ausgefällte kondensierte Eisen(III)phosphat von der Reaktionslösung abtrennt.

In dem erfindungsgemäßen Verfahren können die Ausgangsstoffe (Eisenverbindungen, elementares Eisen) in Pulverform, vorzugsweise mit Korngrößen D50 im Bereich von 0,01 µm bis 300 µm, eingesetzt und direkt mit dem Phosphorsäure enthaltenden wässrigen Medium, vorzugsweise mit verdünnter Phosphorsäure, gemischt und umgesetzt werden. Alternativ können die Ausgangsstoffe oder kann ein Teil der Ausgangsstoffe zunächst über eine Fällung und eventuell anschließendes Glühen frisch erzeugt und danach als Filterkuchen weiter verarbeitet werden. Es entsteht eine durch den Feststoffanteil des Rohstoffs gefärbte bzw. getrübte Aufschlämmung (schwarz bis braun bis rot).

Wenn hierin von wässrigem Lösungsmittel die Rede ist, so umfasst dies Ausführungsformen, die ausschließlich Wasser als flüssiges Medium beinhalten, aber auch solche Ausführungsformen, bei denen das flüssige Medium zu einem vorzugsweise überwiegenden Teil aus Wasser besteht, es aber auch Anteile von mit Wasser mischbaren organischen und/oder ionischen Lösungsmitteln bzw. Flüssigkeiten enthalten kann. Es ist bekannt, dass solche Lösungsmittelzusätze einen Einfluss auf das Kristallwachstum und damit auf die resultierende Morphologie des Produkts haben können.

In dem Phosphorsäure enthaltenden wässrigen Medium kommt es zwischen Fe³⁺ aus dem Eisenrohstoff und dem elementaren Eisen zu einer Redoxreaktion, wobei in einer Komproportionierung lösliches Fe²⁺ gebildet wird. Der Reaktionsansatz erwärmt sich um etwa 2 bis 25°C je nach Rohstoff, wenn die entstehende Reaktionswärme nicht abgeführt wird, was prinzipiell nicht notwendig ist. Nach dem Abklingen der Reaktion wird der Ansatz unter Rühren auf höhere Temperaturen, vorzugsweise unterhalb von 65°C, erwärmt, wobei die eingebrachten Feststoffe je nach Zusammensetzung und Reinheit mehr oder weniger vollständig unter Bildung einer typisch grün gefärbten Fe²⁺-Lösung abreagieren. Die Dauer der Reaktion hängt unter anderem von den eingesetzten Rohstoffen und Konzentrationen ab.

Je nach Reinheit der eingesetzten Feststoffe verbleibt eine mehr oder weniger ausgeprägte Trübung in der Lösung, die durch Verbindungen hervorgerufen wird, die unter den Reaktionsbedingungen unlöslich sind. Dieser verbleibende Feststoffanteil kann durch einfache Filtration, Sedimentation, Zentrifugation oder andere geeignete Mittel abgetrennt werden. Die Auswaagen dieser Feststoffe variieren je nach Wahl der in das Verfahren eingesetzten Ausgangsstoffe, Säurekonzentration und Reaktionstemperatur.

Um weitere Verunreinigungen bzw. unerwünschte Stoffe und Verbindungen aus der Lösung zu entfernen, können der Lösung mit Vorteil definierte Fällungsreagenzien zugesetzt werden. So kann z. B. der Kalziumgehalt in der Lösung durch Zusatz von geringen Mengen Schwefelsäure unter Fällung von Kalziumsulfat reduziert werden. Des Weiteren kann mit Vorteil auch eine zusätzliche elektrolytische Fällung oder Abscheidung von unerwünschten Metallionen aus der Lösung durchgeführt werden.

Ein Vorteil des erfindungsgemäßen Verfahrens besteht darin, dass man als Zwischenprodukt eine homogene phosphorsaure wässrige Eisen(II)-Lösung herstellt, von der man sämtliche als Feststoffe vorliegende oder durch Fällungszusätze in Feststoffe überführbare oder elektrolytisch abscheidbare Verunreinigungen mit einfachen Mitteln abtrennen kann, bevor man das Verfahren zur Herstellung des erfindungsgemäßen Produkts fortsetzt. Das erfindungsgemäße Produkt wird dann nicht mit anderen unlöslichen Verunreinigungen ausgefällt. Das erfindungsgemäße Verfahren erlaubt dadurch gegenüber anderen Verfahren die Herstellung eines Produktes mit hoher Reinheit, ohne dass nachträglich besonders aufwendige Reinigungsverfahren durchgeführt werden müssen.

In einer Ausführungsform des erfindungsgemäßen Verfahrens führt man die Umsetzung der Eisenverbindungen zusammen mit elementarem Eisen in Phosphorsäure enthaltendem wässrigem Medium bei einer Temperatur im Bereich von 10°C bis 90°C, vorzugsweise im Bereich von 20°C bis 75°C, besonders bevorzugt im Bereich von 25°C bis 65°C durch. Bei einer zu niedrigen Temperatur ist die Reaktionsgeschwindigkeit langsam und eventuell unwirtschaftlich. Bei einer zu hohen Temperatur kann es teilweise zu einer vorzeitigen und unerwünschten Ausfällung von Eisen(III)orthophosphat unter anderem aufgrund möglicher Festkörperreaktionen an den in der Suspension enthaltenen festen Ausgangsstoffen kommen. Des Weiteren wird durch zu hohe Temperatur der Ablauf von Nebenreaktionen, wie sie unten beschrieben werden, gefördert.

Zweckmäßigerweise führt man die Umsetzung der Eisenverbindungen zusammen mit elementarem Eisen in Phosphorsäure enthaltendem wässrigem Medium unter intensivem Durchmischen durch, vorzugsweise unter Rühren. Hierfür können alle auf dem Gebiet bekannten Mischer und Rührer, die für einen solchen Verwendungszweck geeignet sind, eingesetzt werden. Es können auch mit Vorteil Leitstrahlmischer, Homogenisatoren, Flußreaktionszellen etc. zur Durchmischung und/oder Bewegung des Reaktionsansatzes verwendet werden.

In einer weiteren Ausführungsform des erfindungsgemäßen Verfahrens führt man die Umsetzung der Eisenverbindungen zusammen mit elementarem Eisen in Phosphorsäure enthaltendem wässrigem Medium für einen Zeitraum von 1 min bis 180 min, vorzugsweise von 5 min bis 120 min, besonders bevorzugt von 20 min bis 90 min durch. Die Umsetzung der Eisenverbindungen zusammen mit elementarem Eisen in Phosphorsäure enthaltendem wässrigem Medium kann selbstverständlich zu jedem Zeitpunkt durch Abtrennen der Feststoffe von der wässrigen Lösung abgebrochen werden, wobei man bei unvollständiger Umsetzung unter Umständen einen Ausbeuteverlust hat.

In dem erfindungsgemäßen Verfahren beträgt die Konzentration der Phosphorsäure in dem wässrigen Medium geeigneterweise 5% bis 85%, vorzugsweise 10% bis 40%, besonders bevorzugt 15% bis 30%, bezogen auf das Gewicht der wässrigen Lösung. Niedrige Phosphorsäurekonzentrationen sind wirtschaftlich von Vorteil, wobei die Reaktion bei zu geringen Konzentrationen sehr langsam ablaufen kann, was unter wirtschaftlichen Gesichtspunkten auch unerwünscht sein kann. Bei hohen Phosphorsäurekonzentrationen, wie beispielsweise über 85%, kann es je nach Feinheit der eingesetzten Eisenverbindungen zu deren Verklumpung kommen, was die Dauer der oben beschriebenen Komproportionierungsreaktion zwischen Fe³⁺ und elementarem Eisen beträchtlich erhöht.

In einer Nebenreaktion zwischen dem elementaren Eisen und der Phosphorsäure entsteht Wasserstoffgas, das aus Sicherheitsgründen gezielt abgeführt werden muss. Diese Nebenreaktion lässt sich nicht unterdrücken, so dass stets ein stöchiometrischer Überschuss an elementarem Eisen gegenüber der Menge eingesetzt werden sollte, die zur Umsetzung von Fe³⁺ im Eisenrohstoff benötigt wird. Die exakte Menge dieses Überschusses hängt weitgehend von den Reaktionsbedingungen ab, wie der Feinheit bzw. Oberflächenaktivität der eingesetzten Feststoffe, der Temperatur und der Säurekonzentration. Ein Überschuss von wenigen Prozent der stöchiometrischen Menge hat sich in vielen Fällen als ausreichend erwiesen. Bei Temperaturen oberhalb von 40°C wurde ein Anstieg der Geschwindigkeit der Nebenreaktion beobachtet. Oberhalb von 70° C kann eine gleichzeitige Fällung von Eisenorthophosphat einsetzen, so dass man keine homogene Fe²⁺-Lösung erhält. Kommt es zu der bereits oben genannten Verklumpung der Eisenkomponente, reagiert das elementare Eisen weitgehend über die Nebenreaktion ab. Die entsprechenden Stöchiometrien sind daher an die jeweils gewählten Reaktionsbedingungen sowie an die Reaktivität der eingesetzten Rohstoffe anzupassen.

Nach dem Lösen des Eisen(II) aus dem Ausgangsmaterial und dem Umsetzen des Eisen(III) und des elementaren Eisens durch Komproportionierung zu Eisen(II) und der oben beschriebenen Entfernung gegebenenfalls vorhandener Verunreinigungen wird der erhaltenen phosphorsauren Fe²⁺-Lösung Oxidationsmittel zugesetzt, um Eisen(II) in der Lösung zu oxidieren. Wesentlich ist dabei, dass die Oxidationsbedingungen so gewählt werden, dass keine Eisen(III)phosphate ausgefällt werden. Dies erreicht man, indem man die Temperatur der Reaktionslösung während der Zugabe des Oxidationsmittels durch Kühlung der Reaktionslösung und/oder durch Einstellen der Zugabegeschwindigkeit des Oxidationsmittels im Bereich von 10°C bis ≤ 60°C hält. Da es sich bei der Oxidationsreaktion um eine exotherme Reaktion handelt, kann die Temperatur durch Kühlung des Reaktionsansatzes im vorgenannten Bereich gehalten werden. Gleichzeitig oder alternativ kann aber auch durch Einstellen der Zugabegeschwindigkeit des Oxidationsmittels ein zu starker Temperaturanstieg des Reaktionsansatzes verhindert werden, insbesondere können durch langsame Zugabe und gleichzeitiges Rühren starke lokale Überhitzungen verhindert werden. Steigt die Temperatur während der Oxidationsreaktion zu hoch an, besteht die Gefahr, dass Eisenorthophosphat ausfällt, was unerwünscht ist.

Die Oxidation wird fortgesetzt, bis im Wesentlichen der gesamte Anteil an Eisen(II) zu Eisen(III) oxidiert wurde und sich kein Eisen(II) mehr nachweisen lässt bzw. eine vorgegebene Eisen(II)-Konzentration unterschritten ist. Während der Oxidation ändert sich die Farbe der Lösung von grün (aufgrund der Fe²⁺-Ionen) nach rosa (aufgrund der nach der Oxidation vorliegenden Fe³⁺-Ionen). Für den Nachweis von Eisen(II) in der wässrigen Lösung stehen dem Fachmann bekannte Schnelltests (z. B. Teststäbchen oder Teststreifen) zur Verfügung, deren Genauigkeit für die Zwecke der vorliegenden Erfindung ausreichend ist.

In einer bevorzugten Ausführungsform des erfindungsgemäßen Verfahrens ist das Oxidationsmittel, welches zugegeben wird, um Eisen(II) in der Lösung zu oxidieren, eine wässrige Lösung von Wasserstoffperoxid (H₂O₂). Die Wasserstoffperoxidlösung hat bevorzugt eine Konzentration von 15 bis 50 Gew.-%, besonders bevorzugt 30 bis 40 Gew.-%.

In alternativen Ausführungsformen des erfindungsgemäßen Verfahrens ist das Oxidationsmittel, welches zugegeben wird, um Eisen(II) in der Lösung zu oxidieren, ein unter Luft, reinem Sauerstoff oder Ozon ausgewähltes gasförmiges Medium, welches in die wässrige Lösung eingeblasen wird.

Nach dem Abschluss der Oxidationsreaktion gibt man der erhaltenen wässrigen Fe³⁺-Lösung Polyphosphate in der Form von Polyphosphorsäure oder ihrer Salze als Feststoffe oder als wässrige Lösung zu, wobei kondensiertes Eisen(III)phosphat gemäß der Erfindung ausfällt. Der Begriff Polyphosphorsäure im Sinne der vorliegenden Erfindung umfasst auch Pyrophosphorsäure.

In einer Ausführungsform des erfindungsgemäßen Verfahrens gibt man der wässrigen Fe³⁺-Lösung die Polyphosphate in der Form einer wässrigen Lösung von Polyphosphorsäure zu, wobei die Polyphosphorsäure vorzugsweise einen P₂O₅-Gehalt im Bereich von 74 bis 80 Gew.-%, besonders bevorzugt einen P₂O₅-Gehalt im Bereich von 76 bis 78 Gew.-%, aufweist. Die Polyphosphorsäure kann aber auch unverdünnt zugegeben werden, was jedoch mit Handhabungsschwierigkeiten verbunden sein kann, da die unverdünnte Polyphosphorsäure in Abhängigkeit von ihrem P₂O₅-Gehalt und der Temperatur sehr viskos sein kann, insbesondere bei Raumtemperatur. Verdünnt man die Polyphosphorsäure, so muss man die einsetzende Hydrolyse der kondensierten Einheiten beachten. Dem Fachmann sind Hydrolyseraten in Abhängigkeit von Temperatur und pH-Wert allgemein bekannt und zugänglich. Das Resultat einer vollständigen Hydrolyse von Polyphosphorsäure ist Orthophosphorsäure.

Alternativ oder ergänzend zu der Zugabe von Polyphosphorsäure gibt man in einer weiteren Ausführungsform des erfindungsgemäßen Verfahrens die Polyphosphate in der Form von Salzen der Polyphosphorsäure als wässrige Lösung oder als Feststoff zu, vorzugsweise in der Form von Natrium- und/oder Kaliumsalzen der Polyphosphorsäure, besonders bevorzugt in der Form von saurem Natriumpyrophosphat (Na₂H₂P₂O₇; SAPP) und/oder von Tetranatriumpyrophosphat (Na₄P₂O₇; TSPP).

Durch die Zugabe von Polyphosphaten in Form der freien Polyphosphorsäure oder ihrer Salze kommt es zur Bildung der kondensierten Phosphate des Eisens in Form von Feststoffen, die aus der Lösung ausfallen und sich durch geeignete Technologien von der flüssigen Phase abtrennen lassen. Das Abtrennen der kondensierten Eisen(III)phosphate von der wässrigen Lösung erfolgt vorzugsweise durch Filtration, Sedimentation, Zentrifugieren oder Kombinationen der vorgenannten Trennverfahren.

In einer bevorzugten Ausführungsform des erfindungsgemäßen Verfahrens wäscht man das ausgefällte kondensierte Eisen(III)phosphat nach dem Abtrennen von der Reaktionslösung wenigstens einmal oder mehrmals mit Wasser, vorzugsweise mit entionisiertem Wasser, bis eine Dispersion von 1 Gew.% des gewaschenen kondensierten Eisen(ttt)phosphats in entionisiertem Wasser eine Leitfähigkeit < 1000 µS/cm, vorzugsweise < 500 µS/cm, besonders bevorzugt < 300 µS/cm aufweist.

Durch den Waschvorgang werden Kontaminationen, wie beispielsweise überschüssiges Phosphat aus der phosphorsauren Fe(III)-Lösung) und/oder über die Polyphosphatsalze eingebrachte Kationen, z. B. Na⁺ aus TSPP oder SAPP, von der Oberfläche der gefällten und abgetrennten Feststoffe entfernt. Die gemessene Leitfähigkeit der Dispersion des Produktes nimmt daher mit zunehmender Entfernung solcher Kontaminationen ab.

In einer weiteren bevorzugten Ausführungsform des erfindungsgemäßen Verfahrens wird das ausgefällte und vorzugsweise gewaschene kondensierte Eisen(III)phosphat entwässert oder getrocknet. Dies erfolgt bei erhöhter Temperatur und/oder unter vermindertem Druck. Alternativ kann das kondensierte Eisen(III)phosphat nach der Abtrennung und dem Waschen auch vorteilhaft in feuchter Form als Filterkuchen oder Dispersion mit Feststoffgehalten von 1 bis 90 Gew.-%, je nach möglicher oder gewünschter Effizienz des Entwässerungsschrittes, weiterverarbeitet werden.

Das erfindungsgemäße Verfahren zur Herstellung von kondensiertem Eisen(III)phosphat hat gegenüber anderen bekannten Verfahren neben der erzielbaren hohen Reinheit des Endproduktes auch einige ökologische und ökonomische Vorteile. Die nach der Abtrennung von kondensiertem Eisen(III)phosphat zurückbleibende Mutterlauge enthält im wesentlichen keine verunreinigenden Umsetzungsprodukte, wie beispielsweise Sulfate oder Chloride, die bei den bekannten Verfahren nach dem Stand der Technik zurückbleiben, bei denen Eisensulfat oder Eisenchlorid als Ausgangsmaterial eingesetzt werden. Mutterlauge aus dem Verfahren gemäß der vorliegenden Erfindung kann daher durch Zugabe konzentrierter Phosphorsäure und/oder durch Erhöhen der Konzentrationen in der Lösung durch Erwärmen wieder auf die gewünschte Konzentration eingestellt werden und ist somit vollständig in den Prozess rückführbar. Die Verluste an P₂O₅ und Fe lassen sich somit minimieren, was die Herstellung unter ökonomischen und ökologischen Gesichtspunkten besonders vorteilhaft macht. Zudem entfällt eine spezielle Behandlung des Abwassers bzw. Waschwassers, um Anionen (z.B. Sulfat, Chlorid, Nitrat, Citrat) zu entfernen. Dies spart Kosten und vermeidet unerwünschte Abfälle.

Durch das erfindungsgemäße Verfahren lassen sich gezielt Produkte mit verschiedenem mittlerem Kondensationsgrad herstellen. Die folgende Tabelle 1 beschreibt die resultierenden Fe/P₂O₅-Verhältnisse für verschiedene Anteile von Eisenpyrophosphat (FePP) und Eisenorthophosphat (FOP) in einem Produkt. Für ein reines FePP mit Kondensationsgrad 2 ergibt sich theoretisch ein Verhältnis von etwa 0,525. Anteile von nicht kondensierten Orthophosphaten (wie z.B. FOP) erhöhen das Verhältnis. Dies bedeutet wiederum, dass Produkte mit kleinerem Fe/P₂O₅-Verhältnis einen höheren mittleren Kondensationsgrad aufweisen müssen und Polyphosphate des Eisens mit einem mittleren Kondensationsgrad > 2 enthalten.

**Tabelle 1 - Fe/P₂O₅-Verhältnisse für verschiedene Anteile von FePP und FOP**

| **FePP (%)** | **FOP (%)** | **Fe/P₂O₅** |
|---|---|---|
| 100% | 0% | 0,5248 |
| 99% | 1% | 0,5269 |
| 98% | 2% | 0,5291 |
| 97% | 3% | 0,5312 |
| 96% | 4% | 0,5334 |
| 95% | 5% | 0,5355 |
| 94% | 6% | 0,5377 |
| 93% | 7% | 0,5399 |
| 92% | 8% | 0,5421 |
| 91% | 9% | 0,5442 |
| 90% | 10% | 0,5464 |
| 89% | 11% | 0,5487 |
| 88% | 12% | 0,5509 |
| 87% | 13% | 0,5531 |
| 86% | 14% | 0,5553 |
| 85% | 15% | 0,5576 |
| 84% | 16% | 0,5598 |
| 83% | 17% | 0,5621 |
| 82% | 18% | 0,5644 |
| 81% | 19% | 0,5666 |
| 80% | 20% | 0,5689 |
| 0% | 100% | 0,7868 |

Die Erfindung umfasst auch ein kondensiertes Eisen(III)phosphat der allgemeinen Formel Fe₍ₙ₊₂₎(PₙO₃ₙ₊₁)₃ · xH₂O mit n ≥ 2 und x ≤ 9, welches nach dem hierin beschriebenen Verfahren herstellbar oder hergestellt ist, wobei das Eisen(III)phosphat amorph bzw. röntgenamorph ist.

In einer bevorzugten Ausführungsform der Erfindung weist das erfindungsgemäße kondensierte Eisen(III)phosphat der allgemeinen Formel Fe₍ₙ₊₂₎(PₙO₃ₙ₊₁)₃ · xH₂O mit n ≥ 2 und x ≤ 9 Farbwerte im Lab-Farbraum von L ≥ 93, a ≤ 0,2, b ≤ 11, vorzugsweise L ≥ 95, a ≤ 0, b ≤ 7, auf. Das erfindungsgemäße Produkt zeichnet sich damit durch eine helle, vorzugsweise weiße Farbe aus, was in vielen Anwendungsfällen eine erwünschte Produkteigenschaft ist. Im Gegensatz dazu sind viele kondensierte Eisen(III)phosphate nach dem Stand der Technik gelblich bis braun, weshalb sie für Anwendungen häufig ungeeignet sind, bei denen eine solche Färbung unter anderem aus Gründen der Ästhetik unerwünscht ist, wie beispielsweise bei der Herstellung von Arzneimitteln, Lebensmitteln usw. Darüber hinaus ist die helle Farbe des erfindungsgemäßen Produkts charakteristisch für die hohe Reinheit des Produkts, beispielsweise hinsichtlich Verunreinigungen durch Eisenhydroxid Fe(OH)₃, welches eine braune Farbe besitzt und herstellungsbedingt als Verunreinigung in einigen Produkten nach dem Stand der Technik vorkommt, weshalb diese deutlich dunkler sind als die erfindungsgemäßen Produkte.

In einer weiteren bevorzugten Ausführungsform der Erfindung weist das erfindungsgemäße kondensierte Eisen(III)phosphat eine Zusammensetzung, ausgedrückt in Gew.-% Fe₂O₃, Gew.-% P₂O₅ und Gew.-% H₂O (= Glühverlust, GV), von 34 bis 37 Gew.-% Fe₂O₃, 45 bis 48 Gew.-% P₂O₅ und 15 bis 21 Gew.-% H₂O auf, wobei die Summe der Gewichtsanteile 100 Gew.-% beträgt. Produkte mit Zusammensetzungen innerhalb dieser Bereiche entsprechen nahezu ideal den Konformitätsanforderungen des FCC.

In einer weiteren bevorzugten Ausführungsform der Erfindung weist das erfindungsgemäße kondensierte Eisen(III)phosphat einen Gehalt an Fe von ≥ 20 Gew.-% und ein Fe:P₂O₅-Gewichtsverhältnis von 0,400 bis 0,580 auf. Besonders bevorzugt ist ein Gehalt an Fe von 20 bis 25 Gew.-% und ein Fe:P₂O₅-Gewichtsverhältnis von 0,515 bis 0,540. Produkte mit Zusammensetzungen innerhalb dieser Bereiche entsprechen nahezu ideal den Konformitätsanforderungen des FCC.

In einer weiteren bevorzugten Ausführungsform der Erfindung weist das erfindungsgemäße kondensierte Eisen(III)phosphat einen Chloridgehalt (Cl⁻) und/oder einen Nitratgehalt (NO₃⁻) und/oder einen Sulfatgehalt (SO₄)²⁻ und/oder einen Natriumgehalt (Na⁺) von < 1000 ppm, vorzugsweise < 500 ppm, besonders bevorzugt < 100 ppm, auf. Das erfindungsgemäße kondensierte Eisen(III)phosphat lässt sich nach dem erfindungsgemäßen Verfahren in sehr hoher Reinheit hinsichtlich der vorgenannten Fremdionen herstellen. Zu hohe Gehalte der genannten Fremdionen können bei der späteren Anwendung des Produktes stören, zum Beispiel durch farbliche Veränderung, Beeinflussung des Geschmacks, Verklumpen oder unerwünschte chemische Reaktionen mit anderen Formulierungskomponenten.

Die erfindungsgemäßen kondensierten Eisen(III)phosphate der allgemeinen Formel Fe₍ₙ₊₂₎(PₙO₃ₙ₊₁)₃ · xH₂O mit n ≥ 2 und x ≤ 9 sind amorph bzw. röntgenamorph, d. h. sie liefern im Röntgenbeugungsdiffraktogramm keine Reflexe.

Durch Tempern lassen sich die erfindungsgemäßen kondensierten Eisen(III)phosphate in eine kristalline Phase überführen. Um dies zu untersuchen, wurden erfindungsgemäße Produkte gemäß Beispiel 6 (siehe unten) und kommerzielle Vergleichsprodukte bei Temperaturen von 150 °C, 250 °C, 350 °C, 450 °C, 550 °C bzw. 650 °C für 2 Stunden unter Luftatmosphäre getempert und anschließend ebenfalls unter Luftatmosphäre auf Raumtemperatur abgekühlt. Bei den Tempertemperaturen von 550 °C und 650 °C verfestigten sich die ursprünglich pulverförmigen Proben, so dass sie für die weiteren Untersuchungen mit einem Mörser zerkleinert werden mussten.

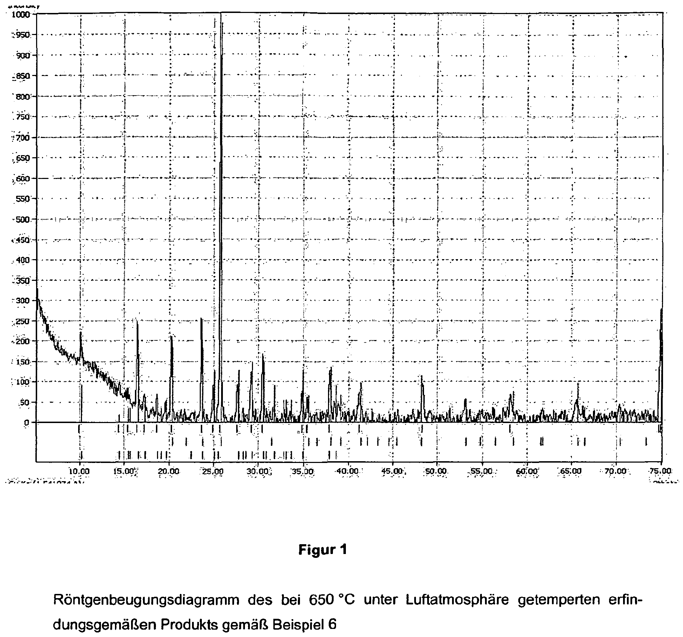

Es wurde gefunden, dass das erfindungsgemäße Produkt erst bei einer Temperatur über 550 °C als wasserfreies FePP kristallisierte. Im Röntgenbeugungsdiagramm waren auch Reflexe von wasserfreiem Eisenorthophosphat (FePO₄) erkennbar, was auf eine teilweise Zersetzung der Ausgangsverbindung unter Bildung flüchtiger Zersetzungsprodukte zurückgeführt wurde. Das erhaltene Röntgenbeugungsdiagramm konnte anhand der PDF-Karten 036-0318 für wasserfreies Fe₄(P₂O₇)₃ und 029-0715 für FePO₄ charakterisiert werden. Das Röntgenbeugungsdiagramm des bei 650 °C getemperten erfindungsgemäßen Produkts ist in Figur 1 wiedergegeben.

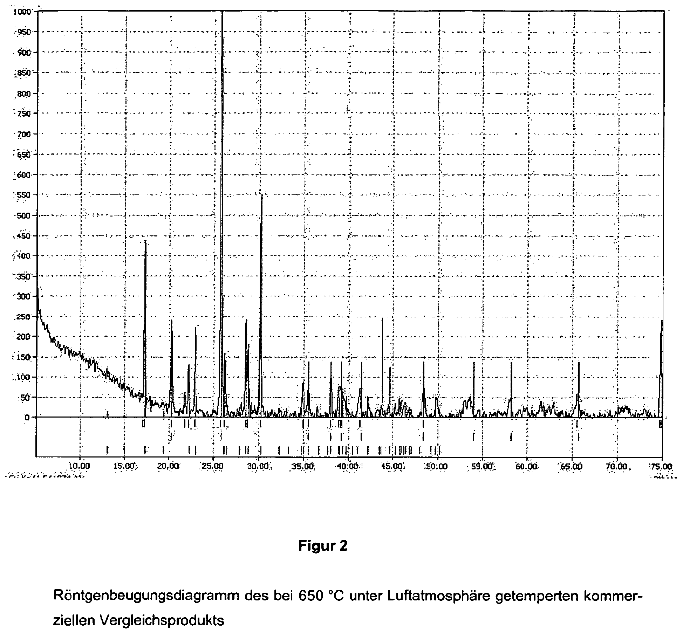

Bei dem kommerziellen Vergleichsprodukt trat eine Kristallisation bereits bei einer Temperatur von 550 °C ein. Das Röntgenbeugungsdiagramm zeigt jedoch nur Reflexe von wasserfreiem NaFeP₂O₇, was auf einen hohen Natriumgehalt im Vergleichsmaterial schließen lässt. Wie bei dem erfindungsgemäßen Produkt zeigen sich auch die Reflexe von wasserfreiem Eisenorthophosphat (FePO₄) aufgrund einer teilweisen Zersetzung der Ausgangsverbindung. Das erhaltene Röntgenbeugungsdiagramm konnte anhand der PDF-Karten 036-1454 für NaFe(P₂O₇) und 017-0837 für FePO₄ charakterisiert werden. Das Röntgenbeugungsdiagramm des bei 650 °C getemperten kommerziellen Vergleichsprodukts ist in Figur 2 wiedergegeben.

In einer bevorzugten Ausführungsform der Erfindung weist das erfindungsgemäße kondensierte Eisen(III)phosphat der allgemeinen Formel Fe₍ₙ₊₂₎(PₙO₃ₙ₊₁)₃ · xH₂O mit n ≥ 2 und x ≤ 9 daher nach Tempern unter Luftatmosphäre bei einer Temperatur von 650°C für einen Zeitraum von 2 Stunden im Pulverröntgenbeugungsspektrum Peaks bei 10,02 ± 0,2, 16,44 ± 0,2, 23,58 ± 0,2, 24,88 ± 0,2, 27,56 ± 0,2, 29,14 ± 0,2, 30,42 ± 0,2 und 34,76 ± 0,2 Grad Zwei-Theta auf, basierend auf CuKa-Strahlung. Dabei handelt es sich um typische Peaks für Fe₄(P₂O₇)₃ gemäß PDF-Karte 036-0318. Des Weiteren weist das erfindungsgemäße kondensierte Eisen(III)phosphat vorzugsweise nach der zuvor beschriebenen Temperbehandlung im Pulverröntgenbeugungsspektrum weitere Peaks bei 20,16 ± 0,2, 25,66 ± 0,2, 37,86 ± 0,2, 41,14 ± 0,2, 47,30 ± 0,2, 48,28 ± 0,2, 52,00 ± 0,2 und 57,10 ± 0,2 Grad Zwei-Theta auf. Dabei handelt es sich um typische Peaks für FePO₄ gemäß PDF-Karte 029-0715.

Die Erfindung umfasst weiterhin die Verwendung von erfindungsgemäßem kondensiertem Eisen(III)phosphat der allgemeinen Formel Fe₍ₙ₊₂₎(PₙO₃ₙ₊₁)₃ · xH₂O mit n ≥ 2 und x ≤ 9 als Nahrungsmitteladditiv, Nahrungsergänzungsmittel, Additiv zur Eisenanreicherung von Nahrungsmitteln für Menschen und Tiere, Pharmazeutikum oder pharmazeutisch aktiver Inhaltsstoff in pharmazeutischen Formulierungen für humanmedizinische und veterinärmedizinische Zwecke, Eisenquelle in chemischen oder biologischen Systemen und/oder zur Herstellung von lithiiertem (Li enthaltendem) Kathodenmaterial für Li-Ionen-Akkumulatoren. Aufgrund seiner hohen Reinheit ist das erfindungsgemäße Produkt vielen bekannten Produkten nach dem Stand der Technik für die vorgenannten Anwendungen deutlich überlegen.

Die Erfindung umfasst weiterhin ein Verfahren zur Herstellung einer stabilisierten Fe³⁺-Lösung, bei dem man
a) eine Fe²⁺-Ionen enthaltende wässrige Lösung herstellt, indem man Eisen(II)-, Eisen(III)- oder gemischte Eisen(II,III)-Verbindungen, ausgewählt unter Hydroxiden, Oxiden, Oxidhydroxiden, Oxidhydraten, Carbonaten und Hydroxidcarbonaten, zusammen mit elementarem Eisen in ein Phosphorsäure enthaltendes wässriges Medium einbringt, wobei Fe²⁺-Ionen in Lösung gebracht werden und Fe³⁺ mit elementarem Fe (in einer Komproportionierungsreaktion) zu gelöstem Fe²⁺ umgesetzt werden,
b) gegebenenfalls vorhandene Feststoffe von der phosphorsauren wässrigen Fe²⁺-Lösung abtrennt,
c) zu der phosphorsauren wässrigen Fe²⁺-Lösung ein Oxidationsmittel zugibt, um Eisen(II) in der Lösung zu oxidieren, wobei die Oxidationsbedingungen so gewählt werden, dass keine Eisen(III)phosphate ausgefällt werden, indem man die Temperatur der Reaktionslösung während der Zugabe des Oxidationsmittels durch Kühlung der Reaktionslösung und/oder durch Einstellen der Zugabegeschwindigkeit des Oxidationsmittels im Bereich von 10°C bis ≤ 60°C hält,

Das Verfahren lässt sich mit Vorteil dadurch ergänzen, dass man vor oder nach dem Abtrennen von gegebenenfalls vorhandenen Feststoffen von der phosphorsauren wässrigen Fe²⁺-Lösung in Stufe b) der erhaltenen phosphorsauren wässrigen Lösung Fällungsreagenzien zugibt, um Feststoffe aus der Lösung auszufällen und/oder in der phosphorsauren wässrigen Lösung gelöste Metalle elektrolytisch aus der Lösung abscheidet.

Die Erfindung umfasst weiterhin eine nach dem vorgenannten Verfahren herstellbare oder hergestellte stabilisierte Fe³⁺-Lösung. Die erfindungsgemäße stabilisierte Fe³⁺-Lösung eignet sich als Vorstufe für die Herstellung verschiedenster Produkte, beispielsweise zur Herstellung von Eisenorthophosphat. Sie zeichnet sich durch eine hohe Reinheit aus, so dass eine aufwendige Aufreinigung vor einer Weiterverarbeitung oder der daraus hergestellten Produkte in der Regel nicht erforderlich ist. Die Lösung ist zudem für einen relativ langen Zeitraum stabil, obwohl Eisen(III)phosphat keine besonders gute Wasserlöslichkeit besitzt. Die hohe Stabilität der Lösung wird auf eine Komplexierung der Fe³⁺-Ionen in der Lösung zurückgeführt.

### Beispiele

### Herstellung der Fe³⁺-Lösung

In einem Becherglas wurden 700 g 75%ige H₃PO₄ und 1580 ml entionisiertes Wasser vorgelegt. Unter Rühren wurden 60 g Fe-Pulver und 75 g Fe₂O₃ zugegeben, wobei sich eine Temperatur von etwa 30 °C einstellte. Anschließend wurde für 2 h bei 60 °C gerührt und danach die resultierende grüne Lösung durch Filtration von gegebenenfalls vorhandenen Schwebstoffen befreit. Die in der erkalteten Lösung vorhandenen Fe²⁺-Ionen wurden unter Rühren durch langsame Zugabe von 220 ml 35 %-iger H₂O₂-Lösung unter Erhalt einer Fe³⁺-Lösung oxidiert, wobei die H₂O₂-Lösung in einer Geschwindigkeit zudosiert wurde, dass die Temperatur nicht über 50 °C stieg.

Die so hergestellte Fe³⁺-Lösung wurde in den nachfolgenden Beispielen eingesetzt. In den Beispielen 1 bis 3 erfolgte die Fällung des Eisen(III)polyphosphats mit TSPP (Beispiel 1), SAPP (Beispiel 2) bzw. einer Kombination aus TSPP und SAPP (Beispiel 3). In den Beispielen 4 bis 7 wurden unterschiedliche Äquivalente einer Polyphosphorsäure mit 76-78 % P₂O₅ eingesetzt.

### Beispiel 1 (TSPP)

87 g Fe³⁺-Lösung (0,056 mol Fe) wurden vorgelegt. 14,89 g TSPP (0,056 mol) wurden in 200 ml vollentsalztem Wasser (VE-Wasser) gelöst und zu der gerührten Fe³⁺-Lösung gegeben. Es fiel ein weißer Niederschlag aus. Der pH-Wert der Lösung betrug 2,3. Der Niederschlag wurde abfiltriert, mit VE-Wasser gewaschen und getrocknet. Der Versuch wurde 2 mal wiederholt.

| Analyse | Wiederholung 1: | Wiederholung 2: |
|---|---|---|
| P₂O₅ (%): | 48,7 | 52,1 |
| Fe (%): | 22,3 | 24,3 |
| GV (%): | 19,4 | 12,3 |
| Fe/P₂O₅: | 0,457 | 0,465 |
| Ausbeute: | 10,7 g | 11,3 g |

### Beispiel 2 (SAPP)

87 g Fe³⁺-Lösung (0,056 mol Fe) wurden vorgelegt. 12,46 g SAPP (0,056 mol) wurden in 120 ml VE-Wasser gelöst und zu der gerührten Fe³⁺-Lösung gegeben. Es fiel ein weißer Niederschlag aus. Der pH-Wert der Lösung wurde mit 50 %-iger NaOH auf 2,5 eingestellt. Der Niederschlag wurde abfiltriert, mit VE-Wasser gewaschen und getrocknet. Der Versuch wurde ohne NaOH-Zugabe wiederholt.

| Analyse | Wiederholung 1 (mit NaOH): | Wiederholung 2 (ohne NaOH): |
|---|---|---|
| P₂O₅ (%): | 51,1 | 52,2 |
| Fe (%): | 21,9 | 23,8 |
| GV (%): | 15,8 | 14,1 |
| Fe/P₂O₅: | 0,429 | 0,455 |
| Ausbeute: | 14,5 g | 14,4 g |

### Beispiel 3 (TSPP + SAPP)

307,6 g einer 3,59 %-igen Fe²⁺-Lösung (0,198 mol Fe) wurden vorgelegt und wie oben beschrieben mit einer 35 %-igen H₂O₂-Lösung vollständig oxidiert. 21,9 g SAPP (0,099 mol) und 26,3 g TSPP (0,099 mol) wurden in 380 ml VE-Wasser gelöst und zu der gerührten Fe³⁺-Lösung gegeben. Es fiel ein weißer Niederschlag aus, der abfiltriert, mit VE-Wasser gewaschen über Nacht bei 55°C unter Erhalt eines weißen Pulvers getrocknet wurde.

| Analyse | |
|---|---|
| P₂O₅ (%): | 53,0 |
| Fe (%): | 21,8 |
| GV (%): | 15,5 |
| Fe/P₂O₅: | 0,411 |
| Ausbeute: | 46,7 g |

### Beispiel 4 (1 Äquivalent Pyrophosphorsäure)

10,0 g H₄P₂O₇ (0,056 mol) wurden in 50 ml VE-Wasser gelöst. 56,8 g der 5,51 %-igen Fe³⁺-Lösung (0,056 mol Fe) wurden zu der gerührten Pyrophosphorsäure-Lösung gegeben. Es fiel ein weißer Niederschlag aus. Der pH-Wert der Lösung betrug 0. Der Niederschlag wurde abfiltriert, mit VE-Wasser gewaschen und getrocknet.

| Analyse | |
|---|---|
| P₂O₅ (%): | 46,0 |
| Fe (%): | 23,2 |
| GV (%): | 20,9 |
| Fe/P₂O₅: | 0,504 |
| Ausbeute: | 8,7 g |

### Beispiel 5 (0,8 Äquivalente Pyrophosphorsäure)

8,0 g H₄P₂O₇ (0,045 mol) wurden in 50 mL VE-Wasser gelöst. 56,8 g der 5,51 %-igen Fe³⁺-Lösung (0,056 mol Fe) wurden zu der gerührten Pyrophosphorsäure-Lösung gegeben. Es fiel ein weißer Niederschlag aus. Der pH-Wert der Lösung betrug 0. Der Niederschlag wurde abfiltriert, mit VE-Wasser gewaschen und getrocknet.

| Analyse | |
|---|---|
| P₂O₅ (%): | 46,3 |
| Fe (%): | 23,7 |
| GV (%): | 20,6 |
| Fe/P₂O₅: | 0,512 |
| Ausbeute: | 11,0 g |

### Beispiel 6 (0,6 Äquivalente Pyrophosphorsäure)

56,8 g der 5,51 %-igen Fe³⁺-Lösung (0,056 mol Fe) wurden vorgelegt. 12,5 g H₄P₂O₇ (0,034 mol) wurden zu der gerührten Fe³⁺-Lösung gegeben. Anschließend wurden 50 ml Wasser zugegeben. Es fiel ein weißer Niederschlag aus. Der pH-Wert der Lösung betrug 0. Der Niederschlag wurde abfiltriert, mit VE-Wasser gewaschen und getrocknet.

| Analyse | |
|---|---|
| P₂O₅ (%): | 45,8 |
| Fe (%): | 24,0 |
| GV (%): | 19,9 |
| Fe/P₂O₅: | 0,524 |
| Ausbeute: | 8,5 g |

Beispiel 6 wurde neun Mal wiederholt (Wiederholungen 6.0 bis 6.8). Die Ergebnisse der Lab-Wertbestimmung sind unten beschrieben.

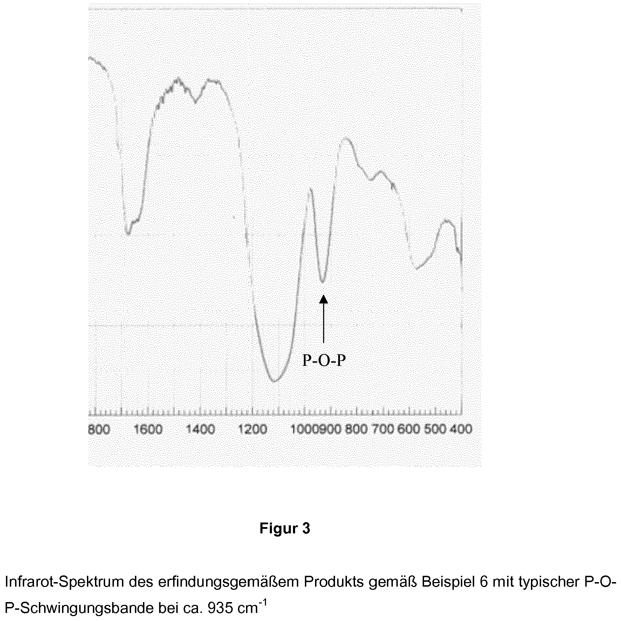

Figur 3 zeigt ein Infrarot-Spektrum des erfindungsgemäßen Produkts gemäß Beispiel 6 (Wiederholung 6.0) mit typischer P-O-P-Schwingungsbande bei ca. 935 cm⁻¹.

### Beispiel 7 (0,5 Äquivalente Pyrophosphorsäure)

100,0 g der 5,51 %-igen Fe³⁺-Lösung (0,1 mol Fe) wurden vorgelegt. 10,4 g H₄P₂O₇ (0,034 mol) wurden in 50 ml Wasser gelöst und zu der gerührten Fe³⁺-Lösung gegeben. Es fiel ein weißer Niederschlag aus. Der pH-Wert der Lösung betrug 0. Es wurden weitere 50 ml Wasser zugegeben. Es fiel weiterer weißer Niederschlag aus. Der Niederschlag wurde abfiltriert, mit VE-Wasser gewaschen und getrocknet.

| Analyse | |
|---|---|
| P₂O₅ (%): | 45,7 |
| Fe (%): | 24,2 |
| GV (%): | 19,7 |
| Fe/P₂O₅: | 0,530 |
| Ausbeute: | 13,6 g |

Die Ausführungsbeispiele zeigen, dass sich mit dem erfindungsgemäßen Verfahren Eisen(III)polyphosphate mit Fe/P₂O₅-Verhältnissen zwischen 0,411 (hoher Kondensationsgrad) und 0,530 herstellen lassen, wobei ein Verhältnis von 0,525 exakt einem reinen FePP entspricht.

Vor allem die Eisen(III)polyphosphate, welche unter Verwendung von Polyphosphorsäure hergestellt wurden, weisen verfahrensbedingt nur geringe Kontaminationen (Spuren) von Alkalimetallen und Anionen wie Sulfat, Chlorid, Nitrat oder Citrat auf.

Die folgende Tabelle 2 zeigt vergleichende Analysen von Eisen(III)phosphaten mit einem Kondensationsgrad von 2. Die kommerziellen Vergleichsmuster wurden aus 3 verschiedenen Quellen bezogen. Die Erfindungsgemäßen Proben sind die Wiederholungen 6.0, 6.5 und 6.8 gemäß Beispiel 6. Die Ergebnisse zeigen, dass keines der kommerziellen Produkte das Fe:P₂O₅-Verhältnis von 0,525 von reinem FePP auch nur annähernd erreicht und somit auch nicht der Kondensationsgrad von etwa 2 bei diesen Produkten vorliegen kann. Zudem weisen die kommerziellen Vergleichsmuster beträchtliche Mengen an Kontaminationen von Sulfat und Natrium auf, wogegen die erfindungsgemäßen Produkte eine sehr hohe Reinheit besitzen.

**Tabelle 2 - Vergleichende Analysen von Eisenpolyphosphaten**

| | kommerzielle Vergleichsmuster | | | erfindungsgemäße Proben gemäß Beispiel 6 | | |
|---|---|---|---|---|---|---|
| | 1 | 2 | 3 | 6.0 | 6.5 | 6.8 |
| Fe [%] | 25 | 24 | 21,9 | 24,1 | 24,7 | 24,4 |
| P₂O₅ [%] | 35,3 | 41,1 | 45 | 45,9 | 46,9 | 45,9 |
| Fe:P₂O₅ | 0,708 | 0,584 | 0,487 | 0,525 | 0,527 | 0,532 |
| As [ppm] | <3 | <3 | <3 | <3 | <3 | <3 |
| Pb [ppm] | <4 | <4 | <4 | <4 | <4 | <4 |
| Cd [ppm] | <1 | <1 | <1 | <1 | <1 | <1 |
| Hg [ppm] | <1 | <1 | <1 | <1 | <1 | <1 |
| SO₄²⁻ [%] | 7,9 | 2,4 | 0,2 | <0,002 | <0,003 | <0,002 |
| Na⁺ [%] | 2,7 | 1,1 | 5,5 | 0,0055 | 0,0055 | 0,0060 |

Bei geeigneter Reaktionsführung lassen sich auch Produkte mit einem Fe/P₂O₅-Verhältnis < 0,411 erhalten.

### Bestimmung von Lab-Werten (L*a*b*)

Der L*a*b*-Farbraum ist ein Messraum, in dem alle wahrnehmbaren Farben enthalten sind. Der Farbraum ist auf Grundlage der Gegenfarbentheorie konstruiert. Eine der wichtigsten Eigenschaften des L*a*b*-Farbmodells ist seine Geräteunabhängigkeit, das heißt, die Farben werden unabhängig von der Art ihrer Erzeugung und Wiedergabetechnik definiert. Die entsprechende deutsche Norm ist *DIN 6174*: "Farbmetrische Bestimmung von Farbmaßzahlen und Farbabständen im angenähert gleichförmigen CIELAB-Farbenraum". Der L*a*b*-Farbraum wird durch ein dreidimensionales Koordinatensystem beschrieben. Die a*-Achse beschreibt den Grün- oder Rotanteil einer Farbe, wobei negative Werte für Grün und positive Werte für Rot stehen. Die b*-Achse beschreibt den Blau- oder Gelbanteil einer Farbe, wobei negative Werte für Blau und positive Werte für Gelb stehen. Die Skalen der a*-Achse und der b*-Achse umfassen einen Zahlenbereich von -150 bis +100 bzw. -100 bis +150, ungeachtet dessen, dass es für einige Werte keine wahrnehmbare Entsprechung gibt. Die L*-Achse beschreibt die Helligkeit (Luminanz) der Farbe mit Werten von 0 bis 100. [Quelle: Wikipedia]

Die folgende Tabelle 3 zeigt die L*a*b*-Werte einer Versuchsreihe (Wiederholungen 6.0 bis 6.8 gemäß Beispiels 6) im Vergleich zu einem kommerziellen Referenzmaterial nach dem Stand der Technik.

**Tabelle 3 - Lab-Werte**

| | 6.0 | 6.1 | 6.2 | 6.3 | 6.4 | 6.5 | 6.6 | 6.7 | 6.8 | Referenz |
|---|---|---|---|---|---|---|---|---|---|---|
| L | 95,7 | 96,1 | 96,2 | 96,4 | 96,4 | 96,4 | 96,1 | 96,3 | 96,3 | 93,9 |
| a | -0,81 | -0,77 | -0,82 | -0,8 | -0,82 | -0,83 | -0,89 | -0,88 | -0,88 | 0,35 |
| b | 5,35 | 5,12 | 5,69 | 5,56 | 5,13 | 5,07 | 5,41 | 5,01 | 5,81 | 11,18 |

Die höheren L-Werte (Luminanz) der Wiederholungen 6.0 bis 6.8 zeigen eine deutlich höhere Helligkeit der erfindungsgemäßen Produkte, und die geringeren b-Werte zeigen eine erheblich geringere Gelbverschiebung. Für den Rot-Grün-Anteil (a-Wert) zeigt sich kein signifikanter Einfluss. Generell wurde festgestellt, dass die erfindungsgemäß hergestellten Produkte alle im Gegensatz zu FePP nach dem Stand der Technik eine nahezu reine weiße Farbe aufweisen.

## Patentansprüche

1. Verfahren zur Herstellung von kondensiertem Eisen(III)phosphat der allgemeinen Formel Fe₍ₙ₊₂₎(PₙO₃ₙ₊₁)₃ · xH₂O mit n ≥ 2 und x ≤ 9, bei dem man
a) eine Fe²⁺-Ionen enthaltende wässrige Lösung herstellt, indem man Eisen(II)-, Eisen(III)- oder gemischte Eisen(II,III)-Verbindungen, ausgewählt unter Hydroxiden, Oxiden, Oxidhydroxiden, Oxidhydraten, Carbonaten und Hydroxidcarbonaten, zusammen mit elementarem Eisen in ein Phosphorsäure enthaltendes wässriges Medium einbringt, wobei Fe²⁺-Ionen in Lösung gebracht werden und Fe³⁺ mit elementarem Fe (in einer Komproportionierungsreaktion) zu gelöstem Fe²⁺ umgesetzt werden,
b) gegebenenfalls vorhandene Feststoffe von der phosphorsauren wässrigen Fe²⁺-Lösung abtrennt,
c) zu der phosphorsauren wässrigen Fe²⁺-Lösung ein Oxidationsmittel zugibt, um Eisen(II) in der Lösung zu oxidieren, wobei die Oxidationsbedingungen so gewählt werden, dass keine Eisen(III)phosphate ausgefällt werden, indem man die Temperatur der Reaktionslösung während der Zugabe des Oxidationsmittels durch Kühlung der Reaktionslösung und/oder durch Einstellen der Zugabegeschwindigkeit des Oxidationsmittels im Bereich von 10°C bis ≤ 60°C hält,
d) nach Abschluss der Oxidationsreaktion der erhaltenen wässrigen Fe³⁺-Lösung Polyphosphate in der Form von Polyphosphorsäure oder ihrer Salze als Feststoffe oder als wässrige Lösung zugibt, wobei kondensiertes Eisen(III)phosphat als Feststoff ausgefällt wird,
e) das ausgefällte kondensierte Eisen(III)phosphat von der Reaktionslösung abtrennt.

2. Verfahren nach Anspruch 1, **dadurch gekennzeichnet, dass** man
f) das ausgefällte kondensierte Eisen(III)phosphat nach dem Abtrennen von der Reaktionslösung wenigstens einmal oder mehrmals mit Wasser, vorzugsweise mit deionisiertem Wasser, wäscht, bis eine Dispersion von 1 Gew.% des gewaschenen kondensierten Eisen(III)phosphats in deionisiertem Wasser eine Leitfähigkeit < 1000 µS/cm, vorzugsweise < 500 µS/cm, besonders bevorzugt < 300 µS/cm aufweist.

3. Verfahren nach Anspruch 1 oder 2, **dadurch gekennzeichnet, dass** man das ausgefällte und vorzugsweise gewaschene kondensierte Eisen(III)phosphat entwässert oder trocknet.

4. Verfahren nach einem der vorangegangenen Ansprüche, **dadurch gekennzeichnet, dass** das Oxidationsmittel, welches man der phosphorsauren wässrigen Fe²⁺-Lösung zugibt, um Eisen(II) in der Lösung zu oxidieren, eine wässrige Lösung von Wasserstoffperoxid (H₂O₂) ist, bevorzugt mit einer Konzentration von 15 bis 50 Gew.-% H₂O₂, besonders bevorzugt 30 bis 40 Gew.-% H₂O₂.

5. Verfahren nach einem der vorangegangenen Ansprüche, **dadurch gekennzeichnet, dass** man vor oder nach dem Abtrennen von gegebenenfalls vorhandenen Feststoffen von der phosphorsauren wässrigen Fe²⁺-Lösung in Stufe b) der in Stufe a) erhaltenen phosphorsauren wässrigen Lösung Fällungsreagenzien zugibt, um Feststoffe aus der Lösung auszufällen und/oder in der phosphorsauren wässrigen Lösung gelöste Metalle elektrolytisch aus der Lösung abscheidet, und / oder
dass man die Umsetzung der Eisenverbindungen zusammen mit elementarem Eisen in Phosphorsäure enthaltendem wässrigem Medium in Stufe a)
- bei einer Temperatur im Bereich von 10°C bis 90°C, vorzugsweise im Bereich von 20°C bis 75°C, besonders bevorzugt im Bereich von 25°C bis 65°C, durchführt und/oder
- unter intensivem Durchmischen durchführt und/oder
- für einen Zeitraum von 1 min bis 180 min, vorzugsweise von 5 min bis 120 min, besonders bevorzugt von 20 min bis 90 min durchführt und / oder
dass die Konzentration der Phosphorsäure in dem Phosphorsäure enthaltenden wässrigen Medium in Stufe a) 5% bis 85%, vorzugsweise 10% bis 40%, besonders bevorzugt 15% bis 30%, beträgt, bezogen auf das Gewicht der wässrigen Lösung.

6. Verfahren nach einem der vorangegangenen Ansprüche, **dadurch gekennzeichnet, dass** man der nach Abschluss der Oxidationsreaktion erhaltenen wässrigen Fe³-Lösung
i) die Polyphosphate in der Form einer wässrigen Lösung von Polyphosphorsäure zugibt, wobei die Polyphosphorsäure vorzugsweise einen P₂O₅-Gehalt im Bereich von 74 bis 80 Gew.-%, besonders bevorzugt einen P₂O₅-Gehalt im Bereich von 76 bis 78 Gew.-%, aufweist, und/oder
ii) die Polyphosphate in der Form von Salzen der Polyphosphorsäure als wässrige Lösung oder als Feststoff zugibt, vorzugsweise in der Form von Natrium- und/oder Kaliumsalzen der Polyphosphorsäure, besonders bevorzugt in der Form von saurem Natriumpyrophosphat (Na₂H₂P₂O₇; SAPP) und/oder von Tetranatriumpyrophosphat (Na₄P₂O₇; TSPP).

7. Kondensiertes Eisen(III)phosphat der allgemeinen Formel Fe₍ₙ₊₂₎(PₙO₃ₙ₊₁)₃ · xH₂O mit n ≥ 2 und x ≤ 9, herstellbar oder hergestellt nach einem der vorangegangenen Ansprüche, wobei das Eisen(III)phosphat amorph bzw. röntgenamorph ist.

8. Kondensiertes Eisen(III)phosphat der allgemeinen Formel Fe₍ₙ₊₂₎(PₙO₃ₙ₊₁)₃ · xH₂O mit n ≥ 2 und x ≤ 9 nach Anspruch 7 **dadurch gekennzeichnet, dass** es Farbwerte im Lab-Farbraum nach Norm DIN 6174 von L ≥ 93, a ≤ 0,2, b ≤ 11, vorzugsweise L ≥ 95, a ≤ 0, b ≤ 7, aufweist.

9. Kondensiertes Eisen(III)phosphat der allgemeinen Formel Fe₍ₙ₊₂₎(PₙO₃ₙ₊₁)₃ · xH₂O mit n ≥ 2 und x ≤ 9 nach einem der Ansprüche 7 oder 8 **dadurch gekennzeichnet, dass**
- das Eisen(III)phosphat eine Zusammensetzung, ausgedrückt in Gew.-% Fe₂O₃, Gew.-% P₂O₅ und Gew.-% H₂O (= Glühverlust, GV), von 34 bis 37 Gew.-% Fe₂O₃, 45 bis 48 Gew.-% P₂O₅ und 15 bis 21 Gew.-% H₂O aufweist, wobei die Summe der Gewichtsanteile 100 Gew.-% beträgt, und/oder
- das Eisen(III)phosphat einen Gehalt an Fe von ≥ 20 Gew.-% und ein Fe:P₂O₅-Gewichtsverhältnis von 0,400 bis 0,580, vorzugsweise einen Gehalt an Fe von 20 bis 25 Gew.-% und ein Fe:P₂O₅-Gewichtsverhältnis von 0,515 bis 0,540 aufweist.

10. Kondensiertes Eisen(III)phosphat der allgemeinen Formel Fe₍ₙ₊₂₎(PₙO₃ₙ₊₁)₃ · xH₂O mit n ≥ 2 und x ≤ 9 nach einem der Ansprüche 7 bis 9, **dadurch gekennzeichnet, dass** es einen Chloridgehalt (Cl⁻) und/oder einen Nitratgehalt (NO₃⁻) und/oder einen Sulfatgehalt (SO₄)²⁻ und/oder einen Natriumgehalt (Na⁺) von < 1000 ppm, vorzugsweise < 500 ppm, besonders bevorzugt < 100 ppm, aufweist.

11. Kondensiertes Eisen(III)phosphat der allgemeinen Formel Fe₍ₙ₊₂₎(PₙO₃ₙ₊₁)₃ · xH₂O mit n ≥ 2 und x ≤ 9 nach einem der Ansprüche 7 bis 10, **dadurch gekennzeichnet, dass** das Eisen(III)phosphat nach Tempern unter Luftatmosphäre bei einer Temperatur von 650°C, für einen Zeitraum von 2 Stunden im Pulverröntgenbeugungsspektrum Peaks bei 10,02 ± 0,2, 16,44 ± 0,2, 23,58 ± 0,2, 24,88 ± 0,2, 27,56 ± 0,2, 29,14 ± 0,2, 30,42 ± 0,2 und 34,76 ± 0,2 Grad Zwei-Theta aufweist, basierend auf CuKa-Strahlung, und vorzugsweise weitere Peaks bei 20,16 ± 0,2, 25,66 ± 0,2, 37,86 ± 0,2, 41,14 ± 0,2, 47,30 ± 0,2, 48,28 ± 0,2, 52,00 ± 0,2 und 57,10 ± 0,2 Grad Zwei-Theta aufweist.

12. Verwendung von kondensiertem Eisen(III)phosphat der allgemeinen Formel Fe₍ₙ₊₂₎(PₙO₃ₙ₊₁)₃ · xH₂O mit n ≥ 2 und x ≤ 9 nach einem der Ansprüche 7 bis 11 als Nahrungsmitteladditiv, Nahrungsergänzungsmittel, Additiv zur Eisenanreicherung von Nahrungsmitteln für Menschen und Tiere, Pharmazeutikum oder pharmazeutisch aktiver Inhaltsstoff in pharmazeutischen Formulierungen für humanmedizinische und veterinärmedizinische Zwecke, Eisenquelle in chemischen oder biologischen Systemen und/oder zur Herstellung von lithiiertem (Li enthaltendem) Kathodenmaterial für Li-Ionen-Akkumulatoren.

13. Verfahren zur Herstellung einer stabilisierten Fe³⁺-Lösung, bei dem man
a) eine Fe²⁺-Ionen enthaltende wässrige Lösung herstellt, indem man Eisen(II)-, Eisen(III)- oder gemischte Eisen(II,III)-Verbindungen, ausgewählt unter Hydroxiden, Oxiden, Oxidhydroxiden, Oxidhydraten, Carbonaten und Hydroxidcarbonaten, zusammen mit elementarem Eisen in ein Phosphorsäure enthaltendes wässriges Medium einbringt, wobei Fe²⁺-Ionen in Lösung gebracht werden und Fe³⁺ mit elementarem Fe (in einer Komproportionierungsreaktion) zu gelöstem Fe²⁺ umgesetzt werden,
b) gegebenenfalls vorhandene Feststoffe von der phosphorsauren wässrigen Fe²⁺-Lösung abtrennt,
c) zu der phosphorsauren wässrigen Fe²⁺-Lösung ein Oxidationsmittel zugibt, um Eisen(II) in der Lösung zu oxidieren, wobei die Oxidationsbedingungen so gewählt werden, dass keine Eisen(III)phosphate ausgefällt werden, indem man die Temperatur der Reaktionslösung während der Zugabe des Oxidationsmittels durch Kühlung der Reaktionslösung und/oder durch Einstellen der Zugabegeschwindigkeit des Oxidationsmittels im Bereich von 10°C bis ≤ 60°C hält,

14. Verfahren nach Anspruch 13, **dadurch gekennzeichnet, dass** man vor oder nach dem Abtrennen von gegebenenfalls vorhandenen Feststoffen von der phosphorsauren wässrigen Fe²⁺-Lösung in Stufe b) der erhaltenen phosphorsauren wässrigen Lösung Fällungsreagenzien zugibt, um Feststoffe aus der Lösung auszufällen und/oder in der phosphorsauren wässrigen Lösung gelöste Metalle elektrolytisch aus der Lösung abscheidet.

15. Stabilisierte Fe³⁺-Lösung, herstellbar oder hergestellt nach Anspruch 13 oder 14.

## Claims

1. Process for the preparation of condensed iron(III) phosphate of the general formula Fe₍ₙ₊₂₎(PₙO₃ₙ₊₁)₃ · xH₂O, where n ≥ 2 and x ≤ 9, in which
a) an aqueous solution comprising Fe²⁺ ions is prepared by introducing iron(II), iron(III) or mixed iron(II,III) compounds selected from hydroxides, oxides, oxide hydroxides, oxide hydrates, carbonates and hydroxide carbonates, together with elemental iron, into an aqueous medium comprising phosphoric acid, Fe²⁺ ions being dissolved and Fe³⁺ being reacted with elemental Fe (in a comproportionation reaction) to give dissolved Fe²⁺,
b) solids present, if appropriate, are separated off from the phosphoric acid aqueous Fe²⁺ solution,
c) an oxidizing agent is added to the phosphoric acid aqueous Fe²⁺ solution in order to oxidize iron(II) in the solution, the oxidation conditions being chosen such that no iron(III) phosphates are precipitated, by keeping the temperature of the reaction solution during the addition of the oxidizing agent in the range of from 10 °C to ≤ 60 °C by cooling the reaction solution and/or by adjusting the rate of addition of the oxidizing agent,
d) after conclusion of the oxidation reaction polyphosphates are added to the resulting aqueous Fe³⁺ solution in the form of polyphosphoric acid or its salts as solids or as an aqueous solution, condensed iron(III) phosphate being precipitated as a solid,
e) the condensed iron(III) phosphate which has precipitated is separated off from the reaction solution.

2. Process according to claim 1, **characterized in that**
f) after being separated off from the reaction solution the condensed iron(III) phosphate precipitated is washed at least once or several times with water, preferably with deionized water, until a dispersion of 1 wt.% of the washed condensed iron(III) phosphate in deionized water has a conductivity of < 1,000 µS/cm, preferably < 500 µS/cm, particularly preferably < 300 µS/cm.

3. Process according to claim 1 or 2, **characterized in that** the precipitated and preferably washed condensed iron(III) phosphate is dewatered or dried.

4. Process according to one of the preceding claims, **characterized in that** the oxidizing agent which is added to the phosphoric acid aqueous Fe²⁺ solution in order to oxidize iron(II) in the solution is an aqueous solution of hydrogen peroxide (H₂O₂), preferably having a concentration of from 15 to 50 wt.% of H₂O₂, particularly preferably 30 to 40 wt.% of H₂O₂.

5. Process according to one of the preceding claims, **characterized in that** before or after the solids present, if appropriate, have been separated off from the phosphoric acid aqueous Fe²⁺ solution in stage b), precipitation reagents are added to the phosphoric acid aqueous solution obtained in stage a), in order to precipitate solids out of the solution, and/or metals dissolved in the phosphoric acid aqueous solution are deposited electrolytically out of the solution, and/or
the reaction of the iron compounds together with elemental iron in an aqueous medium comprising phosphoric acid in stage a)
- is carried out at a temperature in the range of from 10 °C to 90 °C, preferably in the range of from 20 °C to 75 °C, particularly preferably in the range of from 25 °C to 65 °C, and/or
- is carried out with intensive thorough mixing and/or
- is carried out for a period of from 1 min to 180 min, preferably from 5 min to 120 min, particularly preferably from 20 min to 90 min and/or
that the concentration of the phosphoric acid in the aqueous medium comprising phosphoric acid in stage a) is 5 % to 85 %, preferably 10 % to 40 %, particularly preferably 15 % to 30 %, based on the weight of the aqueous solution.

6. Process according to one of the preceding claims, **characterized in that** to the aqueous Fe³⁺ solution obtained after conclusion of the oxidation reaction
i) the polyphosphates are added in the form of an aqueous solution of polyphosphoric acid, the polyphosphoric acid preferably having a P₂O₅ content in the range of from 74 to 80 wt.%, particularly preferably a P₂O₅ content in the range of from 76 to 78 wt.%, and/or
ii) the polyphosphates are added in the form of salts of polyphosphoric acid as an aqueous solution or as a solid, preferably in the form of sodium and/or potassium salts of polyphosphoric acid, particularly preferably in the form of sodium acid pyrophosphate (Na₂H₂P₂O₇; SAPP) and/or of tetrasodium pyrophosphate (Na₄P₂O₇; TSPP).

7. Condensed iron(III) phosphate of the general formula Fe₍ₙ₊₂₎(PₙO₃ₙ₊₁)₃ · xH₂O, where n ≥ 2 and x ≤ 9, which can be prepared or is prepared according to one of the preceding claims, wherein the iron(III)phosphate is amorphous or x-ray amorphous, respectively.

8. Condensed iron(III) phosphate of the general formula Fe₍ₙ₊₂₎(PₙO₃ₙ₊₁)₃ · xH₂O, where n ≥ 2 and x ≤ 9, according to claim 7, **characterized in that** it has colour values in the Lab colour space according to standard DIN 6174 of L ≥ 93, a ≤ 0.2, b ≤ 11, preferably L ≥ 95, a ≤ 0, b ≤ 7.

9. Condensed iron(III) phosphate of the general formula Fe₍ₙ₊₂₎(PₙO₃ₙ₊₁)₃ · xH₂O, where n ≥ 2 and x ≤ 9, according to one of claims 7 or 8, **characterized in that**
- the iron(III) phosphate has a composition, expressed in wt.% of Fe₂O₃, wt.% of P₂O₅ and wt.% of H₂O (= loss on ignition, LOI), of from 34 to 37 wt.% of Fe₂O₃, 45 to 48 wt.% of P₂O₅ and 15 to 21 wt.% of H₂O, the sum of the weight contents being 100 wt.%, and/or
- the iron(III) phosphate has a content of Fe of ≥ 20 wt.% and an Fe:P₂O₅ weight ratio of from 0.400 to 0.580, preferably a content of Fe of from 20 to 25 wt.% and an Fe:P₂O₅ weight ratio of from 0.515 to 0.540.

10. Condensed iron(III) phosphate of the general formula Fe₍ₙ₊₂₎(PₙO₃ₙ₊₁)₃ · xH₂O, where n ≥ 2 and x ≤ 9, according to one of claims 7 to 9, **characterized in that** it has a chloride content (Cl⁻) and/or a nitrate content (NO₃⁻) and/or a sulphate content (SO₄²⁻) and/or a sodium content (Na⁺) of < 1,000 ppm, preferably < 500 ppm, particularly preferably < 100 ppm.

11. Condensed iron(III) phosphate of the general formula Fe₍ₙ₊₂₎(PₙO₃ₙ₊₁)₃ · xH₂O, where n ≥ 2 and x ≤ 9, according to one of claims 7 to 10, **characterized in that**
- after heat treatment under an air atmosphere at a temperature of 650 °C for a period of time of 2 hours, the iron(III) phosphate has peaks in the powder x-ray diffraction spectrum at 10.02 ± 0.2, 16.44 ± 0.2, 23.58 ± 0.2, 24.88 ± 0.2, 27.56 ± 0.2, 29.14 ± 0.2, 30.42 ± 0.2 and 34.76 ± 0.2 degree two-theta, based on CuKα radiation, and preferably further peaks at 20.16 ± 0.2, 25.66 ± 0.2, 37.86 ± 0.2, 41.14 ± 0.2, 47.30 ± 0.2, 48.28 ± 0.2, 52.00 ± 0.2 and 57.10 ± 0.2 degree two-theta.

12. Use of condensed iron(III) phosphate of the general formula Fe₍ₙ₊₂₎(PₙO₃ₙ₊₁)₃ · xH₂O, where n ≥ 2 and x ≤ 9, according to one of claims 7 to 11 as a foodstuffs additive, foodstuffs supplement, additive for enriching foodstuffs for humans and animals with iron, pharmaceutical or pharmaceutically active constituent in pharmaceutical formulations for human medicine and veterinary medicine purposes, source of iron in chemical or biological systems and/or for the production of lithiumated (Li-containing) cathode material for Li ion accumulators.

13. Process for the preparation of a stabilized Fe³⁺ solution, in which
a) an aqueous solution comprising Fe²⁺ ions is prepared by introducing iron(II), iron(III) or mixed iron(II,III) compounds selected from hydroxides, oxides, oxide hydroxides, oxide hydrates, carbonates and hydroxide carbonates, together with elemental iron, into an aqueous medium comprising phosphoric acid, Fe²⁺ ions being dissolved and Fe³⁺ being reacted with elemental Fe (in a comproportionation reaction) to give dissolved Fe²⁺,
b) solids present, if appropriate, are separated off from the phosphoric acid aqueous Fe²⁺ solution,
c) an oxidizing agent is added to the phosphoric acid aqueous Fe²⁺ solution in order to oxidize iron(II) in the solution, the oxidation conditions being chosen such that no iron(III) phosphates are precipitated, by keeping the temperature of the reaction solution during the addition of the oxidizing agent in the range of from 10 °C to ≤ 60 °C by cooling the reaction solution and/or by adjusting the rate of addition of the oxidizing agent.

14. Process according to claim 13, **characterized in that** before or after the solids present, if appropriate, have been separated off from the phosphoric acid aqueous Fe²⁺ solution in stage b), precipitation reagents are added to the phosphoric acid aqueous solution obtained in order to precipitate solids out of the solution, and/or metals dissolved in the phosphoric acid aqueous solution are deposited electrolytically out of the solution.

15. Stabilized Fe³⁺ solution which can be prepared or is prepared according to claim 13 or 14.

## Revendications

1. Procédé de préparation de phosphate de fer (III) condensé de formule générale Fe₍ₙ₊₂₎(PₙO₃ₙ₊₁)₃ . xH₂O avec n ≥ 2 et x ≤ 9, dans lequel
a) on prépare une solution aqueuse contenant des ions Fe²⁺, en ce que l'on introduit des composés de fer(II), de fer(III), ou des composés mixtes de fer(II, III) sélectionnés parmi des hydroxydes, des oxydes, des oxyhydroxydes, des oxydes hydratés, des carbonates et des hydroxycarbonates, avec du fer élémentaire dans un milieu aqueux contenant de l'acide phosphorique, où les ions Fe²⁺ sont mis en solution et Fe³⁺ est mis à réagir avec du Fe élémentaire (dans une réaction de comproportionation) pour donner du Fe²⁺ dissous,
b) on sépare les solides éventuellement présents de la solution aqueuse de Fe²⁺ à l'acide phosphorique,
c) on ajoute à la solution aqueuse de Fe²⁺ à l'acide phosphorique un oxydant pour oxyder le fer(II) en solution, les conditions d'oxydation étant sélectionnées de façon telle qu'aucun phosphate de fer(III) ne précipite, la température de la solution réactionnelle étant maintenue dans la gamme de 10 °C à ≤ 60 °C pendant l'ajout de l'oxydant par refroidissement de la solution réactionnelle et/ou par réglage de la vitesse d'ajout de l'oxydant,
d) on ajoute, après la fin de la réaction d'oxydation de la solution aqueuse de Fe³⁺ obtenue, des polyphosphates sous la forme d'acide polyphosphorique ou de ses sels en tant que solides ou en tant que solution aqueuse, le phosphate de fer(III) condensé précipitant sous la forme d'un solide,
e) on sépare le phosphate de fer(III) condensé précipité de la solution réactionnelle.

2. Procédé selon la revendication 1, **caractérisé en ce que** f) on lave le phosphate de fer(III) condensé précipité après la séparation de la solution réactionnelle au moins une fois ou plusieurs fois avec de l'eau, de préférence avec de l'eau désionisée, jusqu'à ce qu'une dispersion de 1 % en poids du phosphate de fer(III) condensé lavé dans de l'eau désionisée présente une conductibilité < 1 000 µS/cm, de préférence < 500 µS/cm, de façon particulièrement préférée < 300 µS/cm.

3. Procédé selon la revendication 1 ou 2, **caractérisé en ce que** l'on déshydrate ou que l'on sèche le phosphate de fer(III) condensé précipité et de préférence lavé.

4. Procédé selon l'une des revendications précédentes, **caractérisé en ce que** l'oxydant que l'on ajoute à la solution aqueuse de Fe²⁺ à l'acide phosphorique pour oxyder le fer(II) en solution est une solution aqueuse de peroxyde d'hydrogène (H₂O₂), de préférence d'une concentration comprise entre 15 et 50 % en poids de H₂O₂, de façon particulièrement préférée entre 30 et 40 % en poids de H₂O₂.

5. Procédé selon l'une des revendications précédentes, **caractérisé en ce que**, avant ou après la séparation à l'étape b) des solides éventuellement présents de la solution aqueuse de Fe²⁺ à l'acide phosphorique, on ajoute à la solution aqueuse phosphorique obtenue à l'étape a) des réactifs de précipitation pour séparer les solides hors de la solution par précipitation et/ou séparer de la solution par voie électrolytique les métaux dissous dans la solution aqueuse d'acide phosphorique, et/ou **en ce qu'**on met en oeuvre la réaction réalise la conversion des composés de fer, avec le fer élémentaire, dans un milieu aqueux contenant de l'acide phosphorique à l'étape a)
- à une température dans la gamme de 10 °C à 90 °C, de préférence dans la gamme de 20 °C à 75 °C, de façon particulièrement préférée dans la gamme de 25 °C à 65 °C, et/ou
- sous mélange intense, et/ou
- pendant une durée comprise entre 1 min et 180 min, de préférence comprise entre 5 min et 120 min, de façon particulièrement préférée entre 20 min et 90 min, et/ou
**en ce que** la concentration de l'acide phosphorique dans le milieu aqueux contenant de l'acide phosphorique à l'étape a) est comprise entre 5 % et 85 %, de préférence entre 10 % et 40 %, de façon particulièrement préférée entre 15 % et 30 %, par rapport au poids de la solution aqueuse.

6. Procédé selon l'une des revendications précédentes, **caractérisé en ce que** l'on ajoute à la solution aqueuse de Fe³⁺ obtenue après la fin de la réaction d'oxydation,
i) les polyphosphates sous la forme d'une solution aqueuse d'acide polyphosphorique, où l'acide polyphosphorique présente de préférence une teneur en P₂O₅ dans la plage de 74 à 80 % en poids, de façon particulièrement préférée une teneur en P₂O₅ dans la plage de 76 à 78 % en poids, et/ou
ii) les polyphosphates sous la forme de sels de l'acide polyphosphorique en tant que solution aqueuse ou en tant que solide, de préférence sous la forme de sels de sodium et/ou de potassium de l'acide polyphosphorique, de façon particulièrement préférée sous la forme de pyrophosphate de sodium acide (Na₂H₂P₂O₇; SAPP) et/ou de pyrophosphate tétrasodique (Na₄P₂O₇; TSPP).

7. Phosphate de fer(III) condensé de formule générale Fe₍ₙ₊₂₎(PₙO₃ₙ₊₁)₃ . xH₂O avec n ≥ 2 et x ≤ 9, pouvant être préparé ou préparé selon l'une des revendications précédentes, le phosphate de fer(III) étant amorphe ou amorphe aux rayons X.

8. Phosphate de fer(III) condensé de formule générale Fe(ₙ₊₂)(PₙO₃ₙ₊₁)₃ . xH₂O avec n ≥ 2 et x ≤ 9 selon la revendication 7, **caractérisé en ce qu'**il présente des valeurs chromatiques dans l'espace chromatique Lab selon la norme DIN 6174 L ≥ 93, a ≤ 0,2, b ≤ 11, de préférence L ≥ 95, a ≤ 0, b ≤ 7.

9. Phosphate de fer(III) condensé de formule générale Fe₍ₙ₊₂₎(PₙO₃ₙ₊₁)₃ . xH₂O avec n ≥ 2 et x ≤ 9 selon l'une des revendications 7 ou 8, **caractérisé en ce que**
- le phosphate de fer(III) présente une composition, exprimée en % en poids de Fe₂O₃, % en poids de P₂O₅ et % en poids de H₂O (= perte au feu) de 34 à 37 % en poids de Fe₂O₃, 45 à 48 % en poids de P₂O₅ et 15 à 21 % en poids de H₂O, la somme des proportions en poids étant égale à 100 % en poids, et/ou
- le phosphate de fer(III) présente une teneur en Fe de ≥ 20 % en poids et un rapport pondéral Fe:P₂O₅ compris entre 0,400 et 0,580, de préférence une teneur en Fe de 20 à 25 % en poids et un rapport pondéral Fe:P₂O₅ compris entre 0,515 et 0,540.

10. Phosphate de fer(III) condensé de formule générale Fe₍ₙ₊₂₎(PₙO₃ₙ₊₁)₃ . xH₂O avec n ≥ 2 et x ≤ 9 selon l'une des revendications 7 à 9, **caractérisé en ce qu'**il présente une teneur en chlorure (Cl⁻) et/ou une teneur en nitrate (NO₃⁻) et/ou une teneur en sulfate (SO₄)²⁻ et/ou une teneur en sodium (Na⁺) < 1 000 ppm, de préférence < 500 ppm, de façon particulièrement préférée < 100 ppm.

11. Phosphate de fer(III) condensé de formule générale Fe₍ₙ₊₂₎(PₙO₃ₙ₊₁)₃ . xH₂O avec n ≥ 2 et x ≤ 9 selon l'une des revendications 7 à 10, **caractérisé en ce que** le phosphate de fer(III), après mise en température sous atmosphère d'air à une température de 650 °C, pendant une durée de 2 heures, présente dans le spectre de diffraction aux rayons X sur poudre des pics à deux thêta 10,02 ± 0,2, 16,44 ± 0,2, 23,58 ± 0,2, 24,88 ± 0,2, 27,56 ± 0,2, 29,14 ± 0,2, 30,42 ± 0,2 et 34,76 ± 0,2 degrés, sur la base du rayonnement CuKα, et de préférence d'autres pics à deux thêta 20,16 ± 0,2, 25,66 ± 0,2, 37,86 ± 0,2, 41,14 ± 0,2, 47,30 ± 0,2, 48,28 ± 0,2, 52,00 ± 0,2 et 57,10 ± 0,2 degrés deux thêta.

12. Utilisation de phosphate de fer(III) condensé de formule générale Fe(ₙ₊₂)(PₙO₃ₙ₊₁)₃ . xH₂O avec n ≥ 2 et x ≤ 9 selon l'une des revendications 7 à 11 en tant qu'additif alimentaire, complément alimentaire, additif d'enrichissement en fer d'aliments pour l'alimentation humaine et animale, agent pharmaceutique ou composant actif sur le plan pharmaceutique dans des formulations pharmaceutiques à des fins de médecine humaine ou de médecine vétérinaire, source de fer dans des systèmes chimiques ou biologiques et/ou pour fabriquer un matériau de cathode lithié (contenant Li) pour accumulateurs aux ions Li.

13. Procédé de production d'une solution stabilisée de Fe³⁺, dans laquelle
a) on prépare une solution aqueuse contenant des ions Fe²⁺, en ce que l'on introduit des composés de fer(II), de fer(III) ou des composés mixtes de fer(II, III) sélectionnés parmi des hydroxydes, des oxydes, des oxyhydroxydes, des oxydes hydratés, des carbonates et des hydroxycarbonates avec du fer élémentaire dans un milieu aqueux contenant de l'acide phosphorique, où les ions Fe²⁺ sont mis en solution, et Fe³⁺ mis à réagir avec du Fe élémentaire (dans une réaction de comproportionation) pour donner du Fe²⁺ dissous,
b) on sépare les solides éventuellement présents de la solution aqueuse de Fe²⁺ à l'acide phosphorique,
c) on ajoute à la solution aqueuse de Fe²⁺ à l'acide phosphorique un oxydant pour oxyder le fer(II) en la solution, les conditions d'oxydation étant sélectionnées de façon telle qu'aucun phosphate de fer(III) ne précipite, la température de la solution réactionnelle étant maintenue dans la gamme de 10 °C à ≤ 60 °C pendant l'ajout de l'oxydant par refroidissement de la solution réactionnelle et/ou par réglage de la vitesse d'ajout de l'oxydant.

14. Procédé selon la revendication 13, **caractérisé en ce qu'**avant ou après la séparation des solides éventuellement présents de la solution aqueuse de Fe²⁺ à l'acide phosphorique à l'étape b), on ajoute à la solution aqueuse d'acide phosphorique obtenue des réactifs de précipitation pour séparer précipiter les solides de la solution par précipitation et/ou séparer de la solution par voie électrolytique les métaux dissous dans la solution aqueuse d'acide phosphorique.

15. Solution stabilisée de Fe³⁺ pouvant être préparée ou préparée selon la revendication 13 ou 14.
